(19) **Europäisches Patentamt**

**European Patent Office**

**Office européen des brevets**

(11) **EP 1 036 539 A1**

(12) **EUROPEAN PATENT APPLICATION**
published in accordance with Art. 158(3) EPC

(43) Date of publication:
20.09.2000 Bulletin 2000/38

(51) Int. Cl.[7]: **A61B 1/00**, A61B 1/012,
A61B 1/273, A61B 1/31

(21) Application number: 98949238.4

(22) Date of filing: 02.10.1998

(86) International application number:
**PCT/LV98/00006**

(87) International publication number:
**WO 99/17655 (15.04.1999 Gazette 1999/15)**

(84) Designated Contracting States:
**AT BE CH CY DE DK ES FI FR GB GR IE IT LI LU MC NL PT SE**

(30) Priority: **03.10.1997 LV 970190**
**23.09.1998 LV 980188**

(71) Applicant: **Matasova, Vita**
**10439 Berlin (DE)**

(72) Inventor: **MATASOV, Sergey**
**LV-2124 Rigas District (LV)**

(54) **ENDOSCOPE WITH SINGLE-USE CARTRIDGE FOR THE INVAGINATION OF ENDOSCOPIC TUBES**

(57) The present invention pertains to the field of medicine and may be used for carrying out biopsies in the serpinginous channels. This invention more precisely relates to an endoscope that comprises an invagination device in the shape of a compact cylender, wherein said cylinder is separated by a gap from the endoscopic tube and has an outer diameter with periodically narrowing sections as well as an inner diameter with periodically widening sections. The endoscope also includes a rod extraction/introduction system for bending the distal end of the endoscopic tube. This endoscope further includes a cartridge in the shape of a sleeve for receiving the condom of the distal part of the endoscopic tube as well as a spring. One end of the invagination device is turned inside out and is connected to the distal end of the sleeve which is covered with a seal, wherein said invagination device also includes an anal dilator and an endoscopic tube nozzle. The endoscope further includes a table unit, control pedals, a mechanism for supplying the endoscopic tube as wel as a cylinder with a piston for introducing biopsy prongs and for sampling biopsy material in the serpiginous channels.

Fig.1 c

EP 1 036 539 A1

## Description

The invention pertains to the field of medicine, namely to colonoscopy and enteroscopy, but can also be used for industrial endoscopes.

Is known the device under FGR patent No. 3329176, which includes an endoscopic tube, encased in an eversible elastic thin-walled tube which functions as a transporter-invaginator (hereinafter - invaginator) of the first tube. The invaginator in the device patented In FGR is set in long layers parallel to the transported tube. To the drawbacks of this device pertains the inconsequential unreeling of invaginator's layers which is explained by their "sticking together" due to air pressure and inevitable getting of air into spaces between them. Untimely everting of any layer excludes from participation in intubation process other layers located above the everted one.

Is known also the intestinal endoscope under the USSR author's certificate No. 1522466 with an invaginator set in short layers and placed at the right angle with an endoscopic tube, which is transported by it. This endoscope is set as a basis to the present invention and will be taken as a prototype. Endoscope-prototype comprise: - source of light; - source 5 of excessive pressure; - endoscopic tube 3 with eyepiece 1, control bloc 2 with communication branch, stop 11 for spring 10; - invaginator of endoscopic tube 3 which consists of everted part 4 and uneverted part, encased in part 4, at that, the uneverted part of invaginator tightly adjoins to an endoscopic tube and is placed in short layers perpendicularly to it. From the side of uneverted end 7 the invaginator is supported with spring 10, but the place of transition of uneverted part of the invaginator into everted 4 is limited by tip 6. Besides, the endoscope-prototype has: - external seal 13 of tube 3 on which the end 12 of the everted part 4 of invaginator is fixed with ring 16; - rings 8, 9 on the uneverted end 7 of the invaginator, - air-duct 15 with cock 17 for feeding working pressure into cavity 14 of everted part 4 of the invaginator, anal dilator 19. Apart from light and image transmission elements, biopsy channels, channels for gas or liquid supply, an endoscopic tube 3 of the prototype comprises two pairs of closely wound springs containing traction lines which pairwise connects the distal ring of a mechanism for bending the distal end of a tube and rollers for manual extraction of traction lines located in block 2.

The first drawback of the prototype is unreliable functioning of its invaginator - difficulties in introducing endoscopic tube 3 into seal 13 (see 42-53 lines of a.c. No.1522466). The invaginator is to be everted under tip 6, but during invagination the distal part of tube 3 becomes bared. It can be due both to lack of a gap between tube 3 and uneverted part of the invaginator and to a friable structure of the latter, which under the action of air pressure adheres to tube 3. Tube pleats formed during bending of the distal end also prevent free movement of the invaginator along tube 3. As a result the spring is unable to displace the invaginator to tip 6. In addition, the invaginator's end 7, connected with two rings, ensures poor pressurization of cavity 14.

The second drawback of known endoscopes is that bending of its distal end is possible only until a definite number of flexures of an endoscopic tube. Its end is bent by rotating of two rollers each connected to its pair of traction lines. Springs, which comprise traction lines, on the distal end continue channels in the wall of cardan-joint rings. Ends of traction lines are soldered to the distal ring of the cardan mechanism for bending the distal end of the tube. Outward extraction of traction lines from the spring decreases gaps between cardan rings and forms a small radius of a flexure. Herewith, the distal cardan ring pulls the opposite traction line in distal direction, thus ensuring an increase of space between rings. Difference of lengths of big and small half-circumferences of the tube's bend is a product of ⟨⟨π⟩⟩ and diameter of an endoscopic tube. Japanese authors point out that when 3-4 loops are formed, the distal end of an endoscope is blocked, but biopsy forceps continue to function. This difference is explained by L. Aler formula

$$\frac{Q_1}{Q_2} = e^{a.f},$$

where: ⟨⟨$Q_1$⟩⟩ - manual power realizing traction lines extraction; ⟨⟨$Q_2$⟩⟩ - remaining from ⟨⟨$Q_1$⟩⟩ power, attached to a distal cardan ring or cutters of biopsy forceps; ⟨⟨e⟩⟩ - basis of natural logarithm; ⟨⟨$\alpha$⟩⟩ - traction line rotations in radians; ⟨⟨f⟩⟩ - friction index between a traction line and a spring. Under fixed values ⟨⟨$Q_1$⟩⟩ and ⟨⟨f⟩⟩, value ⟨⟨$Q_2$⟩⟩ depends on value ⟨⟨$\alpha$⟩⟩, but for two consecutively connected traction lines of an endoscope the latter is twice as large as for one line of biopsy forceps.

The third drawback of the prototype are problems of its maintenance. For recurrent use an endoscope is washed, disinfected and sterilized. However; there are reported cases of infecting patients with AIDS and other infections after endoscopy. Preparation of the endoscope-prototype for work also includes its assembly. The number of detached parts of this endoscope amounts to 10, but its assembly takes about half an hour. Ergonomics of operating existing endoscopes also impede its mastering. Thus, the left hand holds the control bloc, switches its cocks, rotates handles, which bend and fix the distal end of the tube, but the right hand introduces the tube into the intestine.

It has been practically proved that if an endoscope has more than 3-4 loops, it is impossible to introduce biopsy forceps into it and take bioptate. This is the fourth drawback of the prototype.

The objectives of the invention have been: - increase reliability of invagination of an endoscopic tube; - ensure bending of its distal end in flexuous channels; - make maintenance of an endoscope more convenient; - perform biopsy in flexuous channels. Implementation of these objectives will make colonoscopy available to any physician and make it easier for experienced endoscopists.

These objectives have been achieved by the fact that in the composition of an endoscope, which contains: - source of light; - source of pressure; - biopsy forceps; - an endoscopic tube with the control bloc and communication branch, at that the endoscopic tube contains internally elements for light and image transmission, a channel gas/liquid, a biopsy channel, two pairs of springs with traction lines, which pairwise connect the mechanism for bending the distal end of the endoscopic tube with manual extractors of traction lines located in the control block, but externally a compressed spring placed on tubes distal end, the invaginator, the tip, a mobile seal, an anal dilator, additionally has been included:

- a disposable cartridge for the invagination of an endoscopic tube;
- a system of extractors-intractors of traction lines;
- an essentially changed endoscopic tube;
- a system of introduction and extraction of biopsy forceps;
- a traction line intensifier of biopsy forceps.

The safety of introduction into the intestine and convenience of exploitation of the suggested endoscope is ensured in the first turn by the disposable sterile cartridge which consists of (1st claim): - a shell with projection at its proximal end, comprising: a condom of the distal part of the endoscopic tube fastened at the proximal end with the spring stop; compressed spring; spring distancer in which the distal seal of the endoscopic tube is located, which is joined to an uneverted end of invaginator; fixator of the compressed spring; invaginator in the manner of compact hollow flexible cylinder, which has the gap with the condom and recurrent narrowings of an external and widenings of its internal diameter, at the same time the everted end of invaginator is fastened on the distal end of the shell; - a proximal seal of the endoscopic tube located on the shell; - an anal dilator with the channel in its wall; - the tip of endoscopic tube united with the distal end of condom, which (the tip) has the protective glass, a channel for glass washing and for intestines blowing up, elements for hermetic joining to the endoscopic tube. The compact hollow flexible cylinder of the invaginator is formed of crumpled and tightly compressed in longitudinal and transverse directions short layers of different forms of an eversible thin-walled tube placed at different angles with the longitudinal axis of an endoscopic tube (2nd claim). Moreover, the cartridge for invagination of the endoscopic tube is joined with the mechanism of its introduction in the manner of cylinder with two pistons, which are interconnected with distancers and an elastic tube, but the cavity between them is connected to the source of gas pressure through the pedal cock, in addition the cavity between the proximal seal of the endoscopic tube and distal piston comprises the spring which returns pistons to their home position and through the pedal cock is connected with the source of vacuum (3rd claim).

The system of extractors-intractors of traction lines has the pneumo-hydro-manual drive and creates additional power equal to a few grams at the distal end of traction lines. The system includes the sources of excess pressure and vacuum connected to the cavities of elastic tubes containing liquid and springs with traction lines, in addition tubes are fixed to springs with a thread, but springs are made with steps and ends on the distance from the mechanism for distal end bending, in addition traction lines at the distal end are connected with springs, but in the control block the traction lines are attached to manual extractors-intractors of traction lines connected to elements which ensure synchronous feeding of vacuum into the cavity of manually extracted traction line and feeding of excess pressure into the cavity of an introduced traction line (1st claim). In order to create the additional power the distal end of the tube and traction line it is possible to terminate by a cylinder and piston accordingly, or it is possible to end the tube by an elastic element, for example sylphone, but the traction line connected with its distal end (4th claim). Manual extractors-intractors of traction lines could be made in the manner of a rod, but the sources of pressure and vacuum - of a piston and cylinder, positioned on the rod. An element, which ensures synchronous feeding of vacuum into the cavity of an extracted traction line and pressure into the cavity of an introduced traction line could be a gear mated with cogs of two rods (5th claim). As each of two gears is coupled only with its pair of traction lines, the bending of the tube's end is performed in two stages. The cross-piece with a management lever, whose central part has a movable connection with the body of control bloc, but ends are attached to four rods (6th claim), ensures simultaneous bending of the tube's end in any direction.

A novel endoscopic tube is supplemented with: - internal transverse pleats of its external cover; - two air-ducts, the larger one has a lateral opening into the cavity of the proximal seal of the cartridge for imagination, but the smaller - into the cavity of distal and proximal condoms; - areas for air-tight fixation of condoms' ends; - a proximal condom (1st claim). In addition the control block of the endoscopic tube could be made in the manner of desk, but the cock, feeding the working pressure into the everted part of invaginator can be placed in the pedal (7th claim).

Pneumo-hydro-manual system for intraction and extraction of biopsy forceps includes sources of pressure and vacuum, which are connected through a cock to the cavity of the biopsy channel, the entrance to which is sealed with a seal of biopsy forceps, at the distal end of which there is a piston of the biopsy channel (1st claim).

The biopsy forceps with a pneumo-hidraulic intensifier of traction line contain a flexible hermetic tube, which is connected to sources of pressure and vacuum, but the distal end of the tube and traction line finishes with a cylinder and a piston (1st claim).

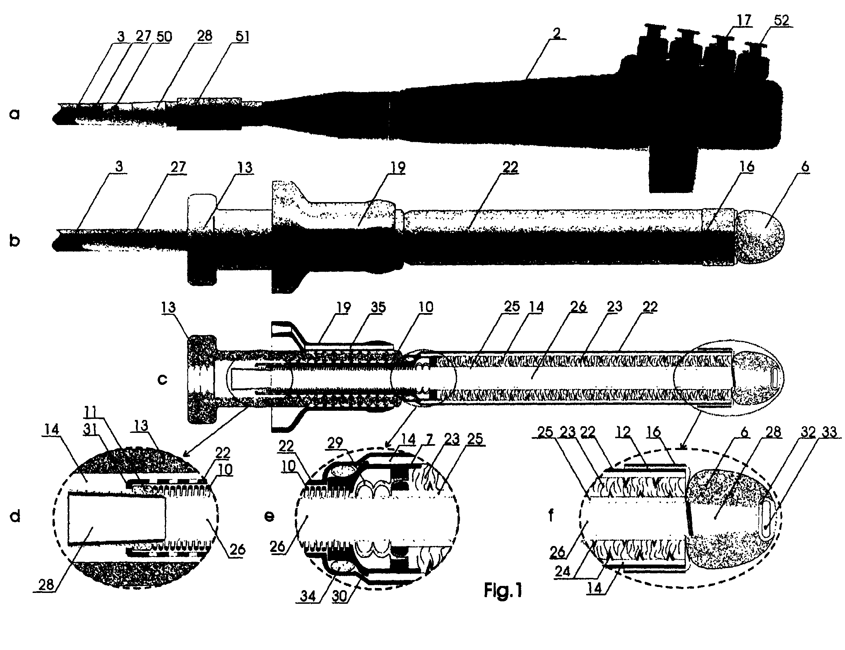

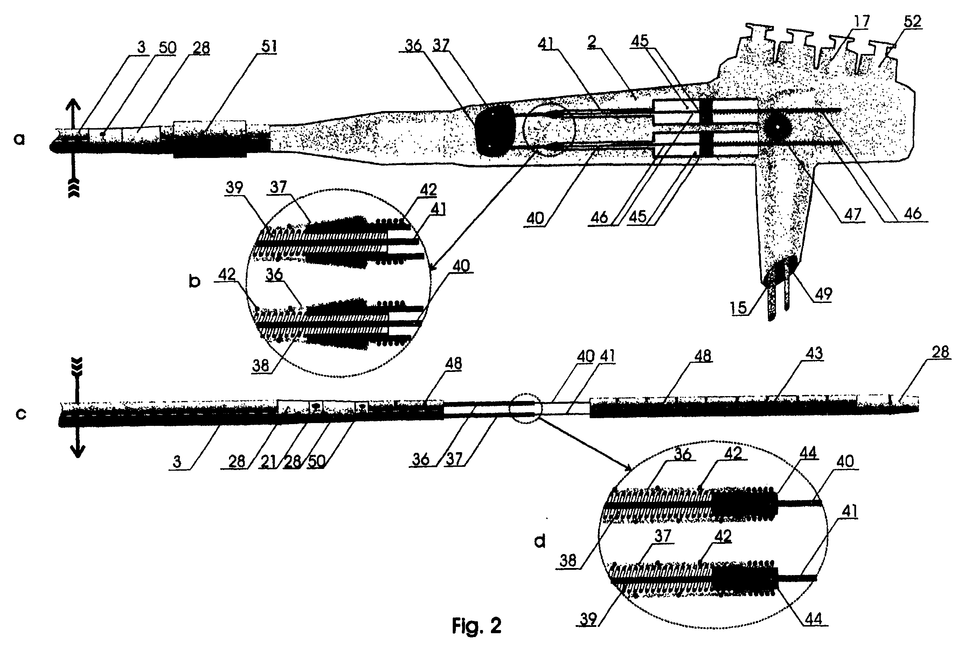

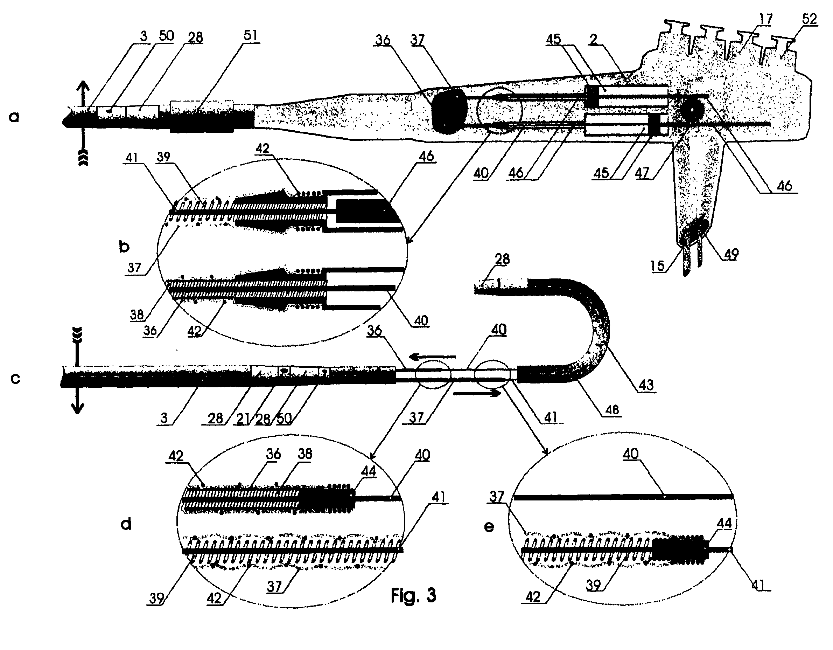

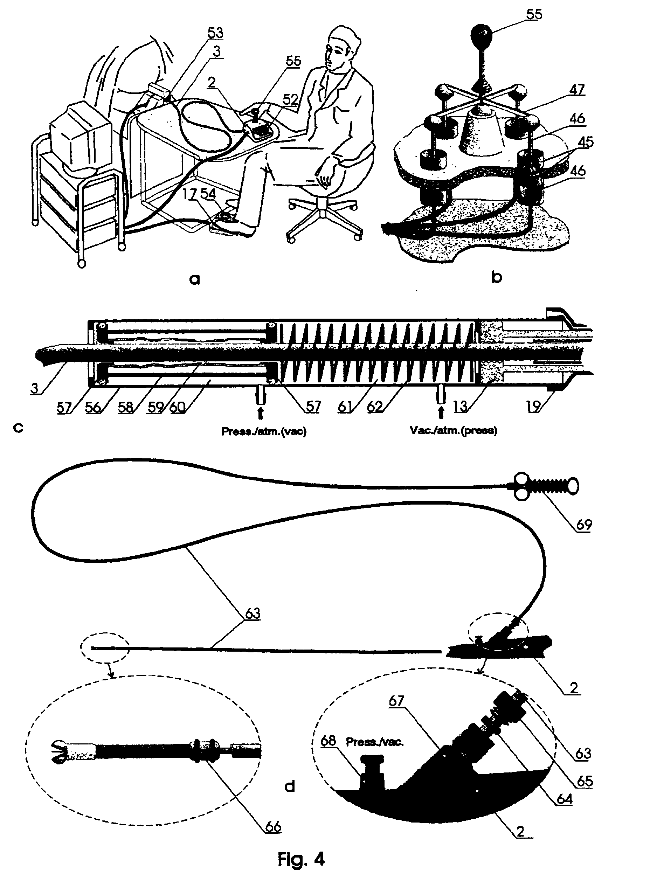

The graphic materials illustrate the essence of invention, where on the fig.1 is represented the endoscope with disposable cartridge for invagination, where: a - handle-shaped control bloc; b - distal part of endoscope with mounted cartridge; c - longitudinal section of cartridge; d, e, f - enlarged fragments of fig.1c. On fig. 2 is represented the system of extraction-intraction of traction lines with pneumo-hydro-manual drive, when the distal end of an endoscope is in direct position, where: a - position of system elements comprised in control bloc; b - enlarged fragment of fig. 2a; c - distal part of endoscope with "bared" system elements (vertical rows show the top-bottom of an endoscopic tube); d - enlarged fragment of fig. 2c. On fig. 3 is represented the system of extraction-intraction of traction lines when the end of an endoscope is bent downwards, where: a - position of elements contained in control bloc; b - enlarged fragment of fig. 3a; c - distal pail of endoscopic tube with "bared" elements (horizontal arrows show the direction of traction lines motion); d, e - enlarged fragments of fig. 3c. On fig. 4 are represented: a - design of new endoscope; b - cross-piece with lever, bending the distal end of endoscope in any direction; c - construction of a mechanism for introduction of endoscopic tube; d - extraction and intraction system of biopsy forceps.

Specification of numerical markings of figs. 1-4 given at the end of the description is practically similar to graphic materials of the prototype. A novel endoscope includes endoscopic tube 3 with control bloc 2 and communication branch. Air-duct 15 and cock 17 positioned on control bloc 2 or in pedal, connect source of working pressure with opening 21 into the cavity of seal 13, which communicates with cavity 14 of shell 22. The distal part of shell 22 is commensurable in relation to length and diameter to uneverted part of invaginator 23, but the proximal part - to the compressed spring 10. Evened end 12 of invaginator 23 is connected to shell 22 by ring 16. Invaginator 23 has narrowings and widenings 24, as well as gap 25 with distal condom 26. Ends of distal 26 and proximal 27 condoms and corresponding to them places of tube 3 have areas 28 for interconnection and hermetization. Seal 29 on end 7 of invaginator 23 separates cavity 14 from cavity 25, which communicates with the intestinal cavity. A distancer 30 prevents deformation of seal 29 by spring 10. Ends of compressed spring 10 are based on distancer 30 and stop 11 at the end 28 of condom 26. Stop 11, in its turn, is positioned on the projection 31 of shell 22. The distal end of condom 26 ends with tip 6 with channels 32 for washing of protective glass 33 and blowing-up of intestines, as well as an element for connection to endoscopic tube 3. On the border of narrow and broad parts of shell 22 there is an area of intermediate diameter with indented elastic ring 34 for fixation of compressed spring 10. Channel 35 of anal dilator 19 is used for decompression of intestines during intubation. In the tube 3, besides the enumerated, there are elastic tubes 36, 37 comprising springs 38, 39 and traction lines 40, 41. Tubes 36, 37 are connected to springs 38, 39 with thread 42. Near mechanism 43 for bending the distal end of tube 3, ends of tubes 36, 37 are closed with plugs 44, which also conned springs 38, 39 with traction lines 40, 41. Proximal ends of tubes 36, 37 are connected with sources 45 of excess pressure and vacuum. Proximal ends of traction lines 40, 41 are connected with their manual extractors-intractors 46, but the latter - with element 47 which ensures synchronous feeding of vacuum into the cavity of traction line 40 which is being extracted and of pressure into the cavity of traction line 41 which is being introduced. Endoscopic tube 3 has internal pleats 48 of external cover, air-duct 49 with two openings 50 for vacuum fixation of condoms 26, 27 to tube 3 and also has removable sleeve gasket 51. Control bloc 2 has cock 52 of air-duct 49. Seal 13 is hermetically connected to mechanism 53 for introduction of endoscopic tube 3. Mechanism 53 for introduction of tube 3 is operated by pedal 54 but lever 55 realizes bending of tubes end. Cylinder 56, two pistons 57, distancers 58 and elastic tube 59 limit cavity 60, which is connected with source of pressure by means of cock in pedal 54. Cavity 61 comprises return spring 62 and is connected with vacuum by means of cock in pedal 54. Seal 64 and nut 65 are mounted on biopsy forceps 63, but piston 66 is positioned at their distal end. Seat for seal 64 and nut 65 is located at entry 67 to biopsy channel, which is positioned with cock 68 on control bloc 2. Sylphone 69, which serves as source of pressure and vacuum in pneumatic intensifier of traction line of biopsy forceps, can be combined with a handle of biopsy forceps 63.

Marks made on condom 27 and tube 3 serves for their correct positioning. Then mechanism 53 is mounted on tube 3 and cartridge for invagination is fixed. Pressing of cock 52 will ensure vacuum fixation of condoms 26, 27 to tube 3. After introduction of seal 13 into cylinder 56 endoscope preparation for work is completed.

After the patent has been placed on an endoscopic table a cartridge is oiled and introduced into the rectum and its ampoule is examined as if with a rigid rectoscope. The pressure in cavity 14 is raised by pressing cock 17 thus freeing distancer 30 from coupling with fixator 34 and shell 22. Thereby spring 10 is released and it is possible to proceed with invagination of tube 3. Eversion of invaginator 23 and introduction of tube 3 into the colon occurs under working pressure in cavity 14 at the moments of pressing pedal 54. During endoscopy intestines are to be distended. Gas into intestines is constantly supplied through gas/liquid channel of tube 3 and through channel 32 of tip 6 thus preventing intestinal content of getting under protective glass 33. Gas evacuation from intestines occurs through channel 35 of anal dilator 19.

Bending of mechanism 43 is accomplished by means of excessive pressure and vacuum sources 45, manual extractors-intractors 46 of traction lines 40, 41 and by means of elements 47 which ensure feeding of vacuum in the cavity of tube 36 which comprises extracted traction line 40, and feeding of excessive pressure in the cavity of tube 37 containing introduced traction line 41. Due to vacuum elastic tube 36 and spring 38 are shortened. Considering, that their distal end is connected with traction line 40, this shortening relieves its manual extraction. Due to pressure in tube 37 the latter and spring 39 elongates towards executive mechanism 43 thus relieving manual infraction of traction line 41. Thread 42 twisted on tubes 36, 37, connects them with springs 38, 39. Thus, vacuum and pressure which shorten and elongate tubes 36,37 and springs 38,39 ensure application of powers to distal ends of traction lines 40 and 41; manual extraction and infraction of traction lines 40, 41 creates synchronous efforts on their proximal ends. Mechanism 43 of tube 3 is bent downwards by the above-mentioned method. During bending of mechanism 43 upwards all above enumerated elements are moved in opposite directions, but bending of mechanism 43 to the left and to the right is implemented by the second pair of traction lines which work similarly. In intermediate positions mechanism 43 is bent by interchangeable application of both pairs of traction lines. Element 47 made in the shape of a cross-piece with lever 55 ensures simultaneous bending of mechanism 43 in any direction.

As during colonoscopy tube 3 repeats all natural flexures of the colon its extubation must not be accelerated. Anal dilator 19 through which extubation is to be conducted eliminates unpleasant sensations caused by this process.

The most practical important version of the invention is a colonoscope with endoscopic tube 3 without biopsy channel. A disposable cartridge ensures an available to all and atraumatic transportation of tube 3 in the colon, condoms 26,27 protect the patient from infections seated in endoscopic tube 3, but tube 3 - from getting contagious during endoscopy. Ergonomy of handling such colonoscope also makes it available to any physician: during endoscopy a physician in sedentary position, watches the screen, presses pedal cock 17 with one foot, pedal 54 with another, the right hand controls lever 55, but in case of necessity washes the protective glass 33 by pressing on the cock with the left hand. Such colonoscope is necessary firstly for family doctors, gastroenterologists and surgeons for regular screening of colon cancer. Having selected "suspicious" patients out-patient physicians will direct them to an in-patient clinic for conducting biopsy and other thorough examination.

For biopsy a cartridge with tip 6, without glass 33 is used. Having exhausted the possibility of manual insertion of forceps 63, it is necessary by means of seal 64 and nut 65 to hermetize entry 67 into the biopsy channel and connect it by means of cock 68 to the source of pressure. Further insertion of forceps 63 is performed by their manual intraction and due to pressure of liquid or gas on piston 66, but extraction - by switching cock 68 in the position 《vacuum》 and manual extraction of forceps 63. Due to location of source 69 of pressure and vacuum of fraction line intensifier in the handle of forceps, taking of bioptate is made as previously - approach of rings ensures movement of the traction line inwards, but detachment - extraction of the traction line.

Specifications of graphic materials' marks on fig. 1-4 and on fig. of the prototype:

1 - eyepiece (fig. of the prototype only);
2 - control bloc with communication branch:
3 - endoscopic tube;
4 - everted part of invaginator (fig. of the prototype only);
5 - source of working pressure in cavity 14 (fig. of the prototype only);
6 - tip of endoscopic tube 3;
7 - uneverted end of invaginator 23;
8,9 - rings at the end 7 of invaginator (fig. of the prototype only);
10 - compressed spring;
11 - stop for spring 10;
12 - everted end of invaginator 23;
13 - proximal seal of tube 3;
14 - cavity of everted part 4 of invaginator 23;
15 - air-duct for feeding working pressure into cavity 14;
16 - ring, fixing end 12 of invaginator 23;
17 - cock of air-duct 15;
18 - manometer (fig. of the prototype only);
19 - anal dilator;
20 - rectum (fig. of the prototype only);
21 - air-duct 15 opening on tube 3;
22 - shell of cartridge for invagination;
23 - invaginator formed in a compact flexible cylinder;
24 - narrowings and widenings of cylinder of invaginator 23;
25 - gap (cavity) between cylinder of invaginator 23 and condom 26;
26 - distal condom of tube 3;
27 - proximal condom of tube 3;
28 - areas on tube 3 and at the ends of condoms 26, 27 for their hermetic connection;
29 - distal seal between tube 3 and end 7 of invaginator 23;

30 - distancer between spring 10 and invaginator 23 comprising seal 29;

31 - projection on shell 22 for stop 11;

32 - channel in tip 6;

33 - protective glass of tip 6;

34 - elastic ring, fixing spring 10 in compressed state;

35 - channel in anal dilator 19;

36 - lower elastic tube of extractor-intractor of traction lines;

37 - upper elastic tube of extractor-intractor of traction lines;

38 - lower spring of extractor-intractor of traction lines;

39 - upper spring of extractor-intractor of traction lines;

40 - lower traction line of extractor-intractor of traction lines;

41 - upper traction line of extractor-intractor of traction lines;

42 - thread fixing elastic tubes 36, 37 to springs 38, 39;

43 - mechanism for bending of distal end of tube 3;

44 - plug dosing tubes 36, 37 and connecting springs 38, 39 with traction lines 40, 41;

45 - sources of pressure and vacuum;

46 - manual extractors-intractors of traction lines 40, 41;

47 - element for extraction-intraction of one or two pairs of traction lines;

48 - pleats of external cover of tube 3;

49 - air-duct into cavity of condoms 26, 27;

50 - distal and proximal openings of air-duct 49 on tube 3;

51 - sleeve gasket;

52 - air-duct 49 cock on control bloc 2;

53 - mechanism for insertion of endoscopic tube 3;

54 - pedal for switching on mechanism 53;

55 - lever of element 47, made in a shape of cross-piece;

56 - cylinder of mechanism 53;

57 - pistons of cylinder 56;

58 - distancers between pistons 57;

59 - elastic tube, attached to pistons 57;

60 - hermetic cavity, enclosed by elastic tube 59 and pistons 57;

61 - hermetic cavity, enclosed by seal 13 and distal piston 57;

62 - spring returning pistons 57 to home position;

63 - biopsy forceps;

64 - seal of entry 67 into biopsy channel;

65 - nut, fixing seal 64;

66 - piston of biopsy forceps;

67 - entry into biopsy channel;

68 - cock feeding pressure or vacuum into biopsy channel;

69 - source of pressure and vacuum connected with cavity of biopsy forceps 63.

**Claims**

1. An endoscope with a disposable cartridges for invagination of an endoscopic tube comprising: - a source of light; - a source of pressure; - biopsy forceps; - an endoscopic tube with a control block and a communication branch containing inwardly light and image transmission elements, a liquid or gas feeding channel, a biopsy channel, two pairs of closely wound springs with traction lines which pairwise connect a mechanism for bending the distal end to manual extractors of traction lines located in a control block, but externally a compressed spring mounted on the tube, an invaginator, a tip, a seal, an anal dilator, **differs in** that the endoscope is supplied with:

■ a disposable cartridge consisting of: - a shell with a projection at its proximal end containing: a condom of the distal part of the endoscopic tube connected to a spring stop; a compressed spring; a spring distancer in which is positioned a distal seal of the endoscopic tube fastened at the uneverted end of the invaginator, a fixator of the compressed spring; the invaginator in the shape of a hollow compact flexible cylinder which has a gap with condom, recurrent narrowings of the external and widenings of internal diameter, at that the everted end of the invaginator is fastened to the distal end of the shell; - a proximal seal of the endoscopic tube mounted on the shell ; - an anal dilator with a channel in its wall; - an endoscopic tube's tip joined to the condom with a protective glass, a channel for glass washing and blowing-up of the intestines, elements for hermetic connection to the endoscopic tube;

■ a system of extractors-intractors of traction lines with a pneumo-hydro-manual drive including the pressure and vacuum sources which are connected to the elastic tubes' cavities, comprising liquid and springs with traction lines, at that the tubes are fixed to the springs with a thread, but the springs are made with steps and terminate at some distance from the mechanism for bending the distal end of the endoscopic tube, at that the traction lines at the distal end are connected to the springs, but in the control block - with the manual extractors-intractors of traction lines, connected with elements for synchronous vacuum feeding into the cavity of the manually extracted traction line and an excess pressure into the cavity of the introduced traction line;

■ an endoscopic tube supplied with: transverse pleats of its external cover turned inwards; two additional air-ducts with cocks, the larger of which communicates with the cavity of the proximal seal of the endoscopic tube through the lateral opening but the smaller one - with the cavity of condoms; areas for air-tight fastening of condoms' ends; a proximal condom;

■ a system for intraction and extraction of biopsy forceps which includes pressure and vacuum sources connected through a cock to a cavity of the biopsy channel, the entry to which hermetized by the seal of biopsy forceps, the distal end of which has a piston of the biopsy channel;

■ a biopsy forceps with traction line intensifier which include a flexible hermetic tube, the cavity of which is connected to pressure and vacuum sources, but the distal end of the traction line and the tube terminates with a piston and a cylinder respectively or the tube ends with an elastic element for instance, a sylphone, but the traction line is connected to its distal end.

2. The endoscope as defined in claim 1 **differs in** that a cylinder of the invaginator is formed of crumpled and tightly compressed in longitudinal and transverse directions short layers of different forms of an eversible thin-walled tube placed at different angles with the longitudinal axis of the endoscopic tube.

3. The endoscope as defined in claim 1 or 2 **differs in** that the proximal end of the cartridge for invagination joins the endoscopic tube introduction mechanism made in the shape of a cylinder with two pistons, which are interconnected with distancers and the elastic tube, but the cavity between them is connected to a source of gas pressure through a pedal cock, at that the cavity between the proximal seal of the endoscopic tube and the distal piston comprises a spring, which returns pistons to their home position and is connected to a source of vacuum through the pedal cock.

4. The endoscope as defined in claim 1, 2 or 3, **differs in** that the distal ends of tubes and traction lines of the system of extractors-intractors of traction lines terminates with a cylinder and a piston, or the tube ends with an elastic element, for instance, a sylphone, but the traction line is connected to its distal end.

5. The endoscope as defined in any preceding claim **differs in** that the manual extractors-intractors of the traction lines are made in the shape of a rod, but pressure and vacuum sources in the shape of a piston and a cylinder, positioned on the rod, but an element for synchronous creating of vacuum in the cavity of the manually extracted traction line and pressure in the cavity of the introduced one is made in the shape of a gear mated with cogs of two rods.

6. The endoscope as defined in any preceding claim **differs in** that the element for simultaneous synchronous feeding of vacuum into the cavity of the manually extracted traction lines and of pressure into the cavity of the introduced traction lines, is made in the shape of a cross-piece with a control lever, the central part of which is movably connected with the body of the control bloc, but ends - with the rods.

7. The endoscope as defined in any preceding claim **differs in** that the control block of the endoscopic tube is made in the shape of a desk one, but the cock for feeding of working pressure into the cavity of the everted part of the invaginator is located in the pedal.

Fig.1

Fig. 2

EP 1 036 539 A1

EP 1 036 539 A1

Fig. 3

Fig. 4

EP 1 036 539 A1

## INTERNATIONAL SEARCH REPORT

International application No.
PCT/LV 98/00006

| A. | CLASSIFICATION OF SUBJECT MATTER |
|---|---|

IPC6.   A61B 1/00, 1/012, 1/273, 1/31

According to International Patent Classification (IPC) or to both national classification and IPC

| B. | FIELDS SEARCHED |
|---|---|

Minimum documentation searched (classification system followed by classification symbols)

IPC6. A61B 1/00, 1/005, 1/012, 1/273, 1/31, 10/00, 17/32, 17/36, 1/045

Documentation searched other than minimum documentation to the extent that such documents are included in the fields searched

Electronic data base consulted during the international search (name of data base and, where practicable, search terms used)

### C.  DOCUMENTS CONSIDERED TO BE RELEVANT

| Category* | Citation of document, with indication, where appropriate, of the relevant passages | Relevant to claim No. |
|---|---|---|
| A | SU 2726050 A (MOSKOVSKY INZHENERNO-FIZICHESKY INSTITUT) 9 October 1970 (09.10.70) | 1-7 |
| A | SU 1097263 A (SPETSIALNOE KONSTRUKTORSKO - TEKHNOLOGICHESKOE BJURO SREDSTV NERAZRUSHAJUSCHEGO KONTROLYA) 15 June 1984 (15.06.84) | 1-7 |
| A | DE 2424749 B2 (OLYMPUS OPTICAL CO.LTD) 21 October 1976 (21.10.76), figures 6,10,11,14,15,16, the claims, column 4, lines 11, 12, 20-23, 28-34 | 1-7 |
| A | DE 1766809 (OLYMPUS OPTICAL CO.,LTD) 31 May 1972 (31.05.72), figures 1;10,12, the claims, column 5, lines 1,2,33-39 | 1-7 |
| A | US 525964 (STM MEDIZINTECHNIK STARNBERG GMBH) 9 November 1993 (09.11.93), figures 1,2, columns 4-8 | 1-7 |

☐ Further documents are listed in the continuation of Box C.    ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 22 December 1998 (22.12. 98) | 27 January 1999 (27.01.99) |

| Name and mailing address of the ISA/ RU | Authorized officer |
|---|---|
| Facsimile No. | Telephone No. |

Form PCT/ISA/210 (second sheet) (July 1992)

12